# EUROPEAN PATENT APPLICATION

(11) **EP 4 361 259 A1**
(43) Date of publication of application: **01.05.2024**
(21) Application number: 22842518.7
(22) Date of filing: 15.07.2022
(51) Int. Cl.: C12N 9/02, C12N 15/52, C12P 23/00, C12N 15/80

(54) **NOVEL BETA-CAROTENE 15,15-OXYGENASE VARIANT AND RETINOID PRODUCTION METHOD USING SAME**

(30) Priority: 15.07.2021 KR 20210093034
(71) Applicant: CJ CheilJedang Corporation, Seoul 04560 (KR)
(72) Inventor: PARK, Hye Min, Seoul 04560 (KR); LEE, Peter, Seoul 04560 (KR); LEE, Dong Pil, Seoul 04560 (KR); KIM, Jae Eung, Seoul 04560 (KR)
(74) Representative: Regimbeau
(86) International application number: PCT/KR2022/010385
(87) International publication number: WO 2023/287256

(57) **Abstract**

The present disclosure relates to a novel *beta*-carotene 15,15'-oxygenase variant, a microorganism including the variant, a method for producing a retinoid using the microorganism, a composition for producing a retinoid, and use of the variant or microorganism for producing a retinoid.

## Description

### [Technical Field]

The present disclosure relates to a microorganism including the variant, a method for producing a retinoid using the microorganism, a composition for producing a retinoid, and use of the variant or microorganism for producing a retinoid.

### [Background Art]

Retinoids, which include vitamin A, a fat-soluble vitamin and an essential nutrient, are currently produced by chemical synthesis and are commercially available. However, for reasons such as supply/demand of raw materials, *etc.,* various studies for producing retinoids based on microbial fermentation (i.e., development of microorganisms capable of high-efficiency production of retinoids and fermentation process technology) are being conducted, for example, a target material-specific approach such as increasing the expression of genes encoding enzymes involved in retinoid biosynthesis or removing genes unnecessary for retinoid biosynthesis. An example of the enzymes involved in the retinoid biosynthesis is beta-carotene 15,15'-oxygenase, *etc.* (US 2020-0277644 A1).

However, studies on beta-carotene 15,15'-oxygenase are insignificant, and there is still a need for a study on the increase of effective vitamin A producing ability in order to meet the increased demand for vitamin A.

### [Disclosure]

### [Technical Problem]

An object of the present disclosure to solve is to provide beta-carotene 15,15'-oxygenase variant and a method for producing a retinoid using the same.

### [Technical Solution]

An object of the present disclosure is to provide a beta-carotene 15,15'-oxygenase variant.

Another object of the present disclosure is to provide a polynucleotide encoding the variant of the present disclosure.

Still another object of the present disclosure is to provide a microorganism which includes the variant of the present disclosure or a polynucleotide encoding the variant.

Still another object of the present disclosure is to provide a method for producing a retinoid which includes culturing a microorganism including the variant of the present disclosure or a polynucleotide encoding the variant in a medium.

Still another object of the present disclosure is to provide a composition for producing a retinoid which includes the variant of the present disclosure, a microorganism including a polynucleotide encoding the variant, or a culture thereof.

Still another object of the present disclosure is to provide use of any one or more of the variant of the present disclosure, a polynucleotide encoding the variant, and a microorganism including the same.

### [Advantageous Effects]

In the case of culturing a microorganism including the beta-carotene 15,15'-oxygenase variant of the present disclosure, it is possible to produce a higher yield of a retinoid compared to a microorganism having an existing unmodified polypeptide.

### [Best Mode for Carrying Out the Invention]

The present disclosure is described in detail as follows. Meanwhile, respective descriptions and embodiments disclosed in the present disclosure may also be applied to other descriptions and embodiments. That is, all combinations of various elements disclosed in the present disclosure fall within the scope of the present disclosure. Additionally, the scope of the present disclosure is not limited by the specific description below. Additionally, a number of papers and patent documents are referenced throughout this specification and their citations are indicated. The contents of the cited documents and patent documents are incorporated by reference into this specification as a whole, and the level of the technical field to which the present disclosure belongs and the content of the present disclosure are more clearly described.

An aspect of the present disclosure provides a beta-carotene 15,15'-oxygenase variant, in which any one or more amino acids among the amino acid corresponding to position 43 from the N-terminus of an amino acid sequence of SEQ ID NO: 1, alanine; the amino acid corresponding to position 58 from the N-terminus of an amino acid sequence of SEQ ID NO: 1, leucine; and the amino acid corresponding to position 74 from the N-terminus of an amino acid sequence of SEQ ID NO: 1, isoleucine, are each substituted with a different amino acid.

The beta-carotene 15,15'-oxygenase variant may be a polypeptide having a beta-carotene 15,15'-oxygenase activity or one in which any one or more amino acids among the amino acids corresponding to positions 43, 58, and 74 from the N-terminus of an amino acid sequence of SEQ ID NO: 1 are substituted with a different amino acid. Specifically, the beta-carotene 15,15'-oxygenase variant may be one in which amino acids at one or more, two or more, or three or more positions of positions 43, 58, and 74 may be substituted, but is not limited thereto.

In the polypeptide having beta-carotene 15,15'-oxygenase activity or beta-carotene 15,15'-oxygenase, which is a parent of the variant, the amino acid corresponding to position 43, from the N-terminus of an amino acid sequence of SEQ ID NO: 1, the amino acid corresponding to position 43 may be alanine; the amino acid corresponding to position 58 may be leucine; and the amino acid corresponding to position 74 may be isoleucine, but the polypeptide is not limited thereto.

In an embodiment, the variant may include one or more substitutions of a substitution of the amino acid corresponding to position 43 from the N-terminus of an amino acid sequence of SEQ ID NO: 1 with an amino acid other than alanine; a substitution of the amino acid corresponding to position 58 from the N-terminus of an amino acid sequence of SEQ ID NO: 1 with an amino acid other than leucine; and a substitution of the amino acid corresponding to position 74 from the N-terminus of an amino acid sequence of SEQ ID NO: 1 with an amino acid other than isoleucine, but the present disclosure is not limited thereto.

The "different amino acid" is not limited as long as it is an amino acid other than the amino acid before substitution. Meanwhile, in the present disclosure, when expressed as "a specific amino acid is substituted", it is apparent that the amino acid is substituted with an amino acid that is different from the amino acid before substitution, even if it is not described as being substituted with a different amino acid.

The substitution with a different amino acid may be a substitution with a nonpolar amino acid, a polar amino acid, or a positively charged (basic) amino acid. Specifically, the nonpolar amino acid may be selected from the group consisting of glycine, alanine, valine, leucine, isoleucine, methionine, and proline, but is not limited thereto. The polar amino acid may be used interchangeably with a hydrophilic amino acid, and may be selected from the group consisting of serine, threonine, cysteine, tyrosine, asparagine and glutamine, and the positively charged (basic) amino acid may be selected from the group consisting of arginine, lysine, and histidine, but is not limited thereto.

The variant of the present disclosure may be a variant in which any one or more of the amino acids corresponding to positions 43, 58, and 74 in an amino acid sequence of SEQ ID NO: 1, which is a reference protein, may be substituted with an amino acid different from the amino acid before substitution, among nonpolar amino acids, polar amino acids, or positively charged (basic) amino acids, but is not limited thereto.

In an embodiment, the substitution of the amino acid corresponding to position 43 with a different amino acid may be a substitution with a nonpolar amino acid; the substitution of the amino acid corresponding to position 58 with a different amino acid may be a substitution with a polar amino acid; and the substitution of the amino acid corresponding to position 74 with a different amino acid may be a substitution with a nonpolar amino acid, but is not limited thereto.

The non-polar amino acid may be valine and the polar amino acid may be glutamine, but is not limited thereto.

The different amino acid may be valine or glutamine, but is not limited thereto.

In an embodiment, the variant of the present disclosure may be one, in which the substitution is selected from the group consisting of a substitution of the amino acid corresponding to position 43 of an amino acid sequence of SEQ ID NO: 1 with valine; a substitution of the amino acid corresponding to position 58 of an amino acid sequence of SEQ ID NO: 1 with glutamine; a substitution of the amino acid corresponding to position 74 of an amino acid sequence of SEQ ID NO: 1 with valine; and a combination thereof, but is not limited thereto.

In an embodiment, the variant of the present disclosure may include, from the N-terminus of an amino acid sequence of SEQ ID NO: 1, i) a substitution of the amino acid corresponding to position 43, alanine, with valine; ii) a substitution of the amino acid corresponding to position 58, leucine, with glutamine; iii) a substitution of the amino acid corresponding to position 74, isoleucine, with valine; iv) a substitution of the amino acid corresponding to position 43, alanine, with valine; and a substitution of the amino acid corresponding to position 58, leucine, with glutamine; v) a substitution of the amino acid corresponding to position 58, leucine, with glutamine; and a substitution of the amino acid corresponding to position 74, isoleucine, with valine; vi) a substitution of the amino acid corresponding to position 43, alanine, with valine; and a substitution of the amino acid corresponding to position 74, isoleucine, with valine; or vii) a substitution of the amino acid corresponding to position 43, alanine, with valine; a substitution of the amino acid corresponding to position 58, leucine, with glutamine; and a substitution of the amino acid corresponding to position 74, isoleucine, with valine, but is not limited thereto.

In an embodiment, the variant of the present disclosure may be a polypeptide including an amino acid sequence represented by SEQ ID NO: 3, in which the amino acid corresponding to position 43 of an amino acid sequence of SEQ ID NO: 1, alanine, is substituted with valine; the amino acid corresponding to position 58 of an amino acid sequence of SEQ ID NO: 1, leucine, is substituted with glutamine; and the amino acid corresponding to position 74, isoleucine of an amino acid sequence of SEQ ID NO: 1, with valine, but is not limited thereto.

The variant of the present disclosure may have, include, consist of, or consist essentially of the amino acid sequence represented by SEQ ID NO: 3.

Additionally, the variant of the present disclosure may include an amino acid sequence, in which in the amino acid sequence represented by SEQ ID NO: 3, the amino acid corresponding to position 43 is set as valine, the amino acid corresponding to position 58 is set as glutamine, and the amino acid corresponding to position 74 is set as valine based on the amino acid sequence of SEQ ID NO: 1, and which has homology or identity of at least 70%, 75%, 80%, 85%, 90%, 95%, 96%, 97%, 98%, 99%, 99.5%, 99.7%, or 99.9% or more to the amino acid sequence represented by SEQ ID NO: 3. Additionally, it is also apparent that a variant having an amino acid sequence having deletion, modification, substitution, conservative substitution, or addition in part of the sequence also falls within the scope of the present disclosure if the amino acid sequence has the above homology or identity in addition to each of the set positions of the sequence and exhibits efficacy corresponding to that of the variant of the present disclosure.

Examples thereof include those having sequence addition or deletion that does not alter the function of the variant of the present disclosure at the N-terminus, C-terminus of the amino acid sequence, and/or within the amino acid sequence, naturally occurring mutation, silent mutation, or conservative substitution.

The "conservative substitution" refers to a substitution of one amino acid with another amino acid having similar structural and/or chemical properties. Such amino acid substitution may generally occur based on similarity in the polarity, charge, solubility, hydrophobicity, hydrophilicity, and/or amphipathic nature of the residues. Usually, conservative substitution may hardly affect or not affect the activity of proteins or polypeptides.

As used herein, the "variant" refers to a polypeptide which has an amino acid sequence different from that of the variant before modification by conservative substitution and/or modification of one or more amino acids but maintains the functions or properties. Such variant may generally be identified by modifying one or more amino acids of the amino acid sequence of the polypeptide and evaluating the properties of the modified polypeptide. That is, the ability of the variant may be increased, unchanged, or decreased compared to that of the polypeptide before modification. Additionally, some variants may include variants in which one or more parts such as an N-terminal leader sequence or a transmembrane domain have been removed. Other variants may include variants in which a part of the N-and/or C-terminus has been removed from the mature protein. The term "variant" may be used interchangeably with terms such as modification, modified polypeptide, modified protein, mutant, mutein, and divergent and is not limited thereto as long as it is a term used with the meaning of variation.

Further, the variant may include deletions or additions of amino acids that have a minimal effect on the properties and secondary structure of the polypeptide. For example, a signal (or leader) sequence that is co-translationally or post-translationally involved in the protein translocation may be conjugated to the N-terminus of the variant. The variant may be conjugated with other sequences or linkers so as to be confirmed, purified, or synthesized.

As used herein, the term "homology" or "identity" refers to the degree of similarity between two given amino acid sequences or nucleotide sequences and may be expressed as a percentage. The terms "homology" and "identity" may often be used interchangeably.

The sequence homology or identity of conserved polynucleotides or polypeptides may be determined by a standard alignment algorithm, and default gap penalties established by a program being used may be used together. Substantially, homologous or identical sequences may generally hybridize with each other as a whole or in part under moderate or highly stringent conditions. It is apparent that the hybridization also includes hybridization with a polynucleotide containing common codons or codons considering codon degeneracy in a polynucleotide.

Whether any two polynucleotide or polypeptide sequences have homology, similarity, or identity may be determined by, for example, a known computer algorithm such as the "FASTA" program using default parameters as in Pearson et al. (1988) Proc. Natl. Acad. Sci. USA 85:2444. Alternatively, these may be determined using the Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later) (including GCG program package (Devereux, J., et al., Nucleic Acids Research 12:387 (1984)), BLASTP, BLASTN, FASTA (Atschul, S. F., et al., J. Mol. Biol. 215:403 (1990); Guide to Huge Computers, Martin J. Bishop, ed., Academic Press, San Diego, 1994; and CARILLO et al. (1988) SIAM J Applied Math 48:1073). For example, homology, similarity, or identity may be determined using BLAST of the National Center for Biotechnology Information or ClustalW.

The homology, similarity, or identity between polynucleotides or polypeptides may be determined by comparing sequence information using the GAP computer program as introduced by, for example, Needleman et al. (1970), J Mol Biol. 48:443, as disclosed by Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. The default parameters for the GAP program may include: (1) a binary comparison matrix (containing a value 1 for identity and a value 0 for non-identity) and the weighted comparison matrix of Gribskov et al. (1986) Nucl. Acids Res. 14:6745 as disclosed in Schwartz and Dayhoff, eds., Atlas of Protein Sequence And Structure, National Biomedical Research Foundation, pp. 353-358 (1979) (or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps.

In an embodiment of the present disclosure, the variant of the present disclosure may have the beta-carotene 15,15'-oxygenase enzyme activity. Additionally, the variant of the present disclosure may have activity that increases retinoid producing ability compared to a wild-type polypeptide having the 15,15'-oxygenase activity.

As used herein, the term "beta-carotene 15,15'-oxygenase" refers to the function of catalyzing the action of cleaving one molecule of beta-carotene into two molecules of retinal.

The beta-carotene 15,15'-oxygenase of the present disclosure may be beta-carotene 15,15'-oxygenase or a polypeptide having the beta-carotene 15,15'-oxygenase activity, to which modification is applied for the preparation of the beta-carotene 15,15'-oxygenase variant provided in the present disclosure. Specifically, the beta-carotene 15,15'-oxygenase may be a naturally occurring polypeptide or wild-type polypeptide, may be a mature polypeptide thereof, and may include a variant or functional fragment thereof, but any one may be included without limitation as long as it can be a parent of the beta-carotene 15,15'-oxygenase variant of the present disclosure.

In the present disclosure, the beta-carotene 15,15'-oxygenase may be a polypeptide of SEQ ID NO: 1, but is not limited thereto. Additionally, the beta-carotene 15,15'-oxygenase may be a polypeptide having a sequence identity of about 80%, 85%, 90%, 95%, 96%, 97%, 98%, or 99% or more to the polypeptide of SEQ ID NO: 1, and any one may fall within the scope of the beta-carotene 15,15'-oxygenase without limitation, as long as it has activity identical or corresponding to that of the polypeptide consisting of the amino acid sequence of SEQ ID NO: 1.

Specifically, the beta-carotene 15,15'-oxygenase of the present disclosure may be used interchangeably with BCO or Blh. In the present disclosure, the sequence of the beta-carotene 15,15'-oxygenase may be obtained from NCBI GenBank, which is a known database. Specifically, the beta-carotene 15,15'-oxygenase may be a polypeptide encoded by *blh* and has beta-carotene 15,15'-oxygenase activity, but is not limited thereto.

As used herein, the term "corresponding to" refers to amino acid residues at positions listed in the polypeptide or amino acid residues that are similar, identical, or homologous to those listed in the polypeptide. Identifying the amino acid at the corresponding position may be determining a specific amino acid in a sequence that refers to a specific sequence. As used herein, the "corresponding region" generally refers to a similar or corresponding position in a related protein or reference protein.

For example, after aligning an arbitrary amino acid sequence with SEQ ID NO: 1, based on the same, each amino acid residue of the amino acid sequence may be numbered with reference to the numerical position of the amino acid residue corresponding to the amino acid residue of SEQ ID NO: 1. For example, a sequence alignment algorithm as described in the present disclosure may enable the determination of the position of an amino acid or a position at which modification such as substitution, insertion, and deletion occurs by comparison with that in a query sequence (also referred to as a "reference sequence").

For such alignments, for example, the Needleman-Wunsch algorithm (Needleman and Wunsch, 1970, J. Mol. Biol. 48:443-453), the Needleman program of the EMBOSS package (EMBOSS: The European Molecular Biology Open Software Suite, Rice et al., 2000), Trends Genet. 16:276-277), *etc.* may be used, but are not limited thereto, and a sequence alignment program, a pairwise sequence comparison algorithm, *etc.,* which are known in the art, may be appropriately used.

Another aspect of the present disclosure provides a polynucleotide encoding a variant of the present disclosure.

As used herein, the term "polynucleotide" is a DNA or RNA strand having a certain length or more as a polymer of nucleotides in which nucleotide monomers are connected in a long chain by covalent bonds, and more specifically, refers to a polynucleotide fragment encoding the variant.

In an embodiment, the polynucleotide encoding the variant of the present disclosure may include a nucleotide sequence encoding the amino acid sequence represented by SEQ ID NO: 3. In an embodiment of the present disclosure, the polynucleotide of the present disclosure may have or include a sequence of SEQ ID NO: 4. Additionally, the polynucleotide of the present disclosure may consist of or essentially consist of a sequence of SEQ ID NO: 4.

In the polynucleotide of the present disclosure, various modifications may be made in the coding region as long as the amino acid sequence of the variant of the present disclosure is not changed in consideration of codon degeneracy or codons preferred in organisms that are intended to express the variant of the present disclosure. Specifically, the polynucleotide of the present disclosure may have or include a nucleotide sequence having 70% or more, 75% or more, 80% or more, 85% or more, 90% or more, 95% or more, 96% or more, 97% or more, 98% or more, and less than 100% homology or identity to the sequence of SEQ ID NO: 4, or may consist of or essentially consist of a nucleotide sequence having 70% or more, 75% or more, 80% or more, 85% or more, 90% or more, 95% or more, 96% or more, 97% or more, 98% or more, and less than 100% homology or identity to the sequence of SEQ ID NO: 4, but is not limited thereto.

In particular, in the sequence having the above homology or identity, the codons encoding the amino acid corresponding to position 43 of SEQ ID NO: 3 may be any one of codons encoding valine; the codons encoding the amino acid corresponding to position 58 of SEQ ID NO: 3 may be any one of codons encoding glutamine; and the codons encoding the amino acid corresponding to position 74 of SEQ ID NO: 3 may be any one of codons encoding valine.

Additionally, the polynucleotide of the present disclosure may include a probe that may be prepared from a known gene sequence, for example, a sequence without limitation as long as it is a sequence that is able to hybridize with a complementary sequence to the entirety or a part of the polynucleotide sequence of the present disclosure under stringent conditions. The "stringent conditions" mean conditions that enable specific hybridization between polynucleotides. These conditions are specifically described in documents (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). Examples thereof include a condition in which polynucleotides having higher homology or identity, i.e., polynucleotides having 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or a condition in which washing is performed once, specifically twice to three times at a salt concentration and temperature equivalent to 60°C, 1X SSC, 0.1% SDS, specifically 60°C, 0.1X SSC, 0.1% SDS, and more specifically 68°C, 0.1X SSC, 0.1% SDS, which are washing conditions for conventional Southern hybridization.

Hybridization requires that two nucleic acids have complementary sequences, although mismatches between bases may be possible depending on the stringency of hybridization. The term "complementary" is used to describe the relation between nucleotide bases capable of hybridization with each other. For example, with regard to DNA, adenine is complementary to thymine, and cytosine is complementary to guanine. Therefore, the polynucleotide of the present disclosure may include isolated nucleic acid fragments that are complementary to the entire sequence as well as substantially similar nucleic acid sequences.

Specifically, a polynucleotide having homology or identity to the polynucleotide of the present disclosure may be detected using a hybridization condition including a hybridization step at a Tₘ value of 55°C and the above-described conditions. Additionally, the Tₘ value may be 60°C, 63°C, or 65°C, but is not limited thereto, and may appropriately be adjusted by those skilled in the art according to the purpose.

The appropriate stringency to hybridize the polynucleotide depends on the length and degree of complementarity of the polynucleotide, and the variables are well known in the art (*e.g.*, J. Sambrook *et al.*, *supra*).

Still another aspect of the present disclosure provides a vector including the polynucleotide of the present disclosure. The vector may be an expression vector for expressing the polynucleotide in a host cell, but is not limited thereto.

The vector of the present disclosure may include a DNA construct including a nucleotide sequence of a polynucleotide encoding a target polypeptide which is operably linked to a suitable expression control region (or expression control sequence) so that the target polypeptide may be expressed in a suitable host. The expression control region may include a promoter capable of initiating transcription, any operator sequence for controlling the transcription, a sequence encoding a suitable mRNA ribosome binding site, and a sequence controlling termination of transcription and translation. The vector may be transformed into a suitable host cell, and then replicated or function independently of the host genome, or may be integrated into the genome itself.

The vector used in the present disclosure is not particularly limited, but any vector known in the art may be used. Examples of commonly used vectors include natural or recombinant plasmids, cosmids, viruses, and bacteriophages. For example, pWE15, M13, MBL3, MBL4, IXII, ASHII, APII, t10, t11, Charon4A, Charon21A, *etc.* may be used as a phage vector or cosmid vector, and pDZ system, pBR system, pUC system, pBluescript II system, pGEM system, pTZ system, pCL system, pET system, *etc.* may be used as a plasmid vector. Specifically, pDZ, pDC, pDCM2 (Korean Patent Application Publication No. 10-2020-0136813), pACYC177, pACYC184, pCL, pECCG117, pUC19, pBR322, pMW118, pCC1BAC, pIMR53 vectors, *etc.* may be used.

In an embodiment, a polynucleotide encoding a target polypeptide may be inserted into a chromosome through a vector for intracellular chromosome insertion. The insertion of the polynucleotide into the chromosome may be performed by any method known in the art, for example, homologous recombination, but is not limited thereto. The vector may further include a selection marker for the confirmation of chromosome insertion. The selection marker is for selecting the cells transformed with vectors, *i.e.*, for confirming the insertion of a target nucleic acid molecule, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells can be selected.

As used herein, the term "transformation" means that a vector including a polynucleotide encoding a target polypeptide is introduced into a host cell or microorganism so that the polypeptide encoded by the polynucleotide can be expressed in the host cell. The transformed polynucleotide may be located regardless of the position, either by being inserted into the chromosome of the host cell and located therein or located outside the chromosome as long as it can be expressed in the host cell. Additionally, the polynucleotide includes DNA and/or RNA encoding a target polypeptide. The polynucleotide may be introduced in any form as long as it can be introduced into a host cell and expressed. For example, the polynucleotide may be introduced into a host cell in the form of an expression cassette, which is a gene construct including all elements required for self-expression. The expression cassette may usually include a promoter operably linked to the polynucleotide, a transcription termination signal, a ribosome binding site, and a translation termination signal. The expression cassette may be in the form of an expression vector capable of self-replication. Additionally, the polynucleotide may be introduced into a host cell in its own form and operably linked to a sequence required for expression in the host cell, but is not limited thereto.

Additionally, the term "operably linked" means that the polynucleotide sequence is functionally linked to a promoter sequence that initiates and mediates transcription of the polynucleotide encoding the target variant of the present disclosure.

Still another aspect of the present disclosure provides a microorganism including the variant of the present disclosure or the polynucleotide of the present disclosure.

The microorganism of the present disclosure may include any one or more among the variant of the present disclosure, a polynucleotide encoding the variant, and a vector including the polynucleotide. The microorganism of the present disclosure may include the modified polypeptide of the present disclosure, a polynucleotide encoding the polypeptide, or a vector including the polynucleotide of the present disclosure.

As used herein, the term "microorganism" or "strain" includes all of wild-type microorganisms and naturally or artificially genetically modified microorganisms, and it may be a microorganism in which a specific mechanism is weakened or enhanced due to the insertion of a foreign gene, enhancement or inactivation of the activity of an endogenous gene, *etc.,* and it may be a microorganism including genetic modification for the production of a target polypeptide, protein, or product.

The microorganism of the present disclosure may be a microorganism including any one or more of the variant of the present disclosure, the polynucleotide of the present disclosure, and the vector including the polynucleotide of the present disclosure; a microorganism modified to express the variant of the present disclosure or the polynucleotide of the present disclosure; a microorganism expressing the variant of the present disclosure or the polynucleotide of the present disclosure (*e.g.*, a recombinant microorganism); or a microorganism having the activity of the variant of the present disclosure (*e.g.*, a recombinant microorganism), but is not limited thereto. The microorganism may be a microorganism which endogenously includes polynucleotides encoding lycopene cyclase/phytoene synthase (crtYB) and phytoene desaturase (crtl) proteins or is modified to further include polynucleotides encoding lycopene cyclase/phytoene synthase (crtYB) and phytoene desaturase (crtl) proteins, thereby exhibiting activities of these proteins or a microorganism in which the activities of these proteins are enhanced. Additionally, the microorganism of the present disclosure may be a microorganism, in which the parent strain not having the activities of lycopene cyclase/phytoene synthase (crtYB) and phytoene desaturase (crtl) proteins is modified to thereby exhibit the activities of these proteins. The lycopene cyclase/phytoene synthase or phytoene desaturase may be a protein derived from *Xanthophyllomyces dendrorhous*, but is not limited thereto. In a specific embodiment, the polynucleotide encoding the lycopene cyclase/phytoene synthase or phytoene desaturase may each have or include a sequence of SEQ ID NO: 13 or SEQ ID NO: 14. In the polynucleotide, various modifications may be made in the coding region as long as the amino acid sequence is not changed in consideration of codon degeneracy or codons preferred in microorganisms that are intended to express the variant of the present disclosure. Specifically, the polynucleotide may have or include a nucleotide sequence having 70% or more, 80% or more, 85% or more, 90% or more, 95% or more, 96% or more, 97% or more, 98% or more, and less than 100% homology or identity to the sequence of SEQ ID NO: 13 or SEQ ID NO: 14, or may consist of or essentially consist of a nucleotide sequence having 70% or more, 80% or more, 85% or more, 90% or more, 95% or more, 96% or more, 97% or more, 98% or more, and less than 100% homology or identity to the sequence of SEQ ID NO: 13 or SEQ ID NO: 14, but is not limited thereto.

The microorganism of the present disclosure may have retinoid producing ability or may have retinol producing ability.

In the present disclosure, "retinoid" refers to a material that can act as vitamin A or is related thereto. Specifically, the retinoid may be any one or more selected from the group consisting of retinol, retinal, retinyl ester, and retinoic acid. The microorganism of the present disclosure may be a microorganism capable of producing retinal and retinol, and retinoic acid may be produced by being converted from retinol.

The microorganism of the present disclosure may be a microorganism which naturally has the ability to produce beta-carotene 15,15'-oxygenase or a retinoid, or a microorganism without the ability to produce *beta*-carotene 15,15'-oxygenase or a retinoid, into which the variant of the present disclosure or a polynucleotide encoding the same (or a vector comprising the vector) is introduced and/or in which the retinoid producing ability is conferred, but is not limited thereto.

In an embodiment, the microorganism of the present disclosure may be a cell or microorganism which is transformed with the polynucleotide of the present disclosure or a vector including the polynucleotide encoding the variant of the present disclosure to express the variant of the present disclosure, and for the purpose of the present disclosure, the microorganism of the present disclosure may include any microorganism that includes the variant of the present disclosure to produce a retinoid. For example, the microorganism of the present disclosure may be a recombinant microorganism in which the polynucleotide encoding the variant of the present disclosure is introduced into a natural wild-type microorganism or retinoid-producing microorganism to express the *beta*-carotene 15,15'-oxygenase variant and to have an increased retinoid producing ability. The recombinant microorganism may be a microorganism having an increased retinoid producing ability compared to a natural wild-type microorganism or dual-functional *beta*-carotene 15,15'-oxygenase unmodified microorganism (*i.e.*, a wild-type microorganism expressing *beta*-carotene 15,15'-oxygenase (SEQ ID NO: 1) or a microorganism not expressing a modified protein (the beta-carotene 15,15'-oxygenase variant of SEQ ID NO: 3 or *beta*-carotene 15,15'-oxygenase variants described above), but is not limited thereto. For example, the beta-carotene 15,15'-oxygenase unmodified microorganism, which is a target microorganism where the presence of an increase in retinoid producing ability is compared, may be strain CC08-1023, but is not limited thereto.

In an embodiment, compared to the retinoid producing ability of the parent strain before modification or the unmodified microorganism, the recombinant microorganism with increased retinoid producing ability may have an increased ability of about 1% or more, and specifically about 1% or more, about 2.5% or more, about 5% or more, about 6% or more, about 7% or more, about 8% or more, about 9% or more, about 10% or more, about 11% or more, about 11.5% or more, about 12% or more, about 12.5% or more, about 13% or more, about 13.5% or more, about 14% or more, about 14.5% or more, about 15% or more, about 15.5% or more, about 16% or more, about 16.5% or more, about 17% or more, about 17.5% or more, about 18% or more, about 20% or more, about 22% or more, about 24% or more, about 26% or more, about 28% or more, about 30% or more, about 32% or more, about 34% or more, about 36% or more, about 37% or more, about 38% or more, about 39% or more, about 40% or more, about 41% or more, about 41.5% or more, about 42% or more, about 42.5% or more, about 43% or more, about 43.5% or more, about 44% or more, about 44.5% or more, about 45% or more, about 45.5% or more, or about 46% or more (the upper limit is not particularly limited, and it may be, for example, about 200% or less, about 150% or less, about 100% or less, or about 50% or less), but is not limited thereto as long as recombinant microorganism has an increased amount of + value compared to the retinoid producing ability of the parent strain or unmodified microorganism before modification. In another embodiment, compared to the retinoid producing ability of the parent strain before modification or the unmodified microorganism, the recombinant microorganism with increased retinoid producing ability may have an increased ability of about 1.01 times or more, about 1.02 times or more, about 1.03 times or more, about 1.05 times or more, about 1.06 times or more, about 1.07 times or more, about 1.08 times or more, about 1.09 times or more, about 1.10 times or more, about 1.11 times or more, about 1.12 times or more, about 1.13 times or more, about 1.14 times or more, about 1.15 times or more, about 1.16 times or more, about 1.17 times or more, about 1.18 times or more, about 1.20 times or more, about 1.22 times or more, about 1.24 times or more, about 1.26 times or more, about 1.28 times or more, about 1.30 times or more, about 1.32 times or more, about 1.34 times or more, about 1.36 times or more, about 1.38 times or more, about 1.40 times or more, about 1.41 times or more, about 1.42 times or more, about 1.43 times or more, about 1.44 times or more, about 1.45 times or more, or about 1.46 times or more (the upper limit is not particularly limited, and it may be, for example, about 10 times or less, about 5 times or less, about 3 times or less, or about 2 times or less), but is not limited thereto. As used herein, the term "about" refers to a range which includes all of ±0.5, ±0.4, ±0.3, ±0.2, ±0.1, *etc.,* and includes all of the values that are equivalent or similar to those following the term "about", but the range is not limited thereto.

As used herein, the term "unmodified microorganism" does not exclude strains including mutation that may occur naturally in microorganisms, and it may be a wild-type strain or natural strain itself or may be a strain before the trait is changed by genetic variation due to natural or artificial factors. For example, the unmodified microorganism may be a strain into which the beta-carotene 15,15'-oxygenase variant of the present disclosure described herein is not introduced or has not yet been introduced. The term "unmodified microorganism" may be used interchangeably with "strain before modification", "microorganism before modification", "unmutated strain", "unmodified strain", "unmutated microorganism", or "reference microorganism".

In still another embodiment, the microorganism of the present disclosure may be a microorganism belong to the *Yarrowia* sp., and specifically *Yarrowia lipolytica,* but is not limited thereto.

As used herein, the term "enhancement" with respect to polypeptide activity means that the activity of a polypeptide is increased compared to its endogenous activity. The enhancement may be used interchangeably with terms such as activation, up-regulation, overexpression, increase, *etc.* In particular, activation, enhancement, up-regulation, overexpression, and increase may include both exhibition of activity that was not originally possessed and exhibition of improved activity compared to the endogenous activity or activity before modification. The "endogenous activity" means the activity of a specific polypeptide originally possessed by a parent strain before the change of a trait or an unmodified microorganism, when the trait is changed by genetic mutation due to natural or artificial factors. This may be used interchangeably with "activity before modification". That the activity of a polypeptide is "enhanced", "up-regulated", "overexpressed", or "increased" compared to its endogenous activity means that the activity of the polypeptide is improved compared to the activity and/or concentration (expression level) of a specific polypeptide originally possessed by a parent strain before the change of the trait or an unmodified microorganism.

The enhancement may be achieved through the introduction of a foreign polypeptide or the enhancement of endogenous activity and/or concentration (expression level) of the polypeptide. The enhancement of the activity of a polypeptide may be confirmed by an increase in the degree of activity and the expression level of the corresponding polypeptide or in the amount of the product produced from the corresponding polypeptide.

For the enhancement of the activity of the polypeptide, various methods well known in the art may be applied, and the method is not limited as long as the activity of the target polypeptide can be enhanced compared to that of the microorganism before modification. Specifically, genetic engineering and/or protein engineering well known to those skilled in the art, which are methods routinely conducted in molecular biology, may be used, but the method is not limited thereto (e.g., Sitnicka et al. Functional Analysis of Genes. Advances in Cell Biology. 2010, Vol. 2. 1-16; Sambrook et al. Molecular Cloning 2012; *etc.).*

Specifically, the enhancement of the polypeptide of the present disclosure may be:
1) increase in the intracellular copy number of the polynucleotide encoding the polypeptide;
2) replacement of a gene expression control region on a chromosome encoding the polypeptide with a sequence having strong activity;
3) modification of a nucleotide sequence encoding an initiation codon or 5'-UTR region of a gene transcript encoding the polypeptide;
4) modification of the amino acid sequence of the polypeptide to enhance the activity of the polypeptide;
5) modification of the polynucleotide sequence encoding the polypeptide to enhance the activity of the polypeptide (e.g., modification of the polynucleotide sequence of the polypeptide gene to encode the polypeptide which is modified to enhance the activity of the polypeptide);
6) introduction of a foreign polypeptide exhibiting the activity of the polypeptide or a foreign polynucleotide encoding the same;
7) codon optimization of the polynucleotide encoding the polypeptide;
8) analysis of the tertiary structure of the polypeptide to select and modify or chemically modify the exposed site; or
9) a combination of two or more selected from 1) to 8) above, but is not particularly limited thereto.

More specifically:
1) The increase in the intracellular copy number of the polynucleotide encoding the polypeptide may be achieved by the introduction, into a host cell, of a vector which may replicate and function independently of the host and to which the polynucleotide encoding the corresponding polypeptide is operably linked. Alternatively, the increase may be achieved by the introduction of one copy or two or more copies of the polynucleotide encoding the corresponding polypeptide into the chromosome of a host cell. The introduction into the chromosome may be performed by introducing a vector capable of inserting the polynucleotide into the chromosome of a host cell into the host cell, but is not limited thereto. The vector is as described above.
2) The replacement of a gene expression control region (or expression control sequence) on the chromosome encoding the polypeptide with a sequence exhibiting strong activity may be, for example, occurrence of mutation in a sequence due to deletion, insertion, non-conservative or conservative substitution, or a combination thereof, or replacement with a sequence having stronger activity so that the activity of the expression control region is further enhanced. The expression control region may include, but is not particularly limited to, a promoter, an operator sequence, a sequence encoding a ribosome binding site, a sequence controlling the termination of transcription and translation, *etc.* For example, the replacement may be to replace the original promoter with a strong promoter, but is not limited thereto.
   Examples of known strong promoters include CJ1 to CJ7promoters (U.S. Patent No. US 7662943 B2), lac promoter, trp promoter, trc promoter, tac promoter, lambda phage PR promoter, PL promoter, tet promoter, gapA promoter, SPL7 promoter, SPL13 (sm3) promoter (U.S. Patent No. US 10584338 B2), O2 promoter (U.S. Patent No. US 10273491 B2), tkt promoter, yccA promoter, TEFINt promoter, EXP1 promoter, TEF1 promoter, *etc.,* but is not limited thereto.
3) The modification of a nucleotide sequence encoding an initiation codon or 5'-UTR region of a gene transcript encoding the polypeptide may be, for example, substitution with a nucleotide sequence encoding a different start codon having a higher polypeptide expression rate compared to an endogenous start codon, but is not limited thereto.
4) and 5) The modification of the amino acid sequence or the polynucleotide sequence may be occurrence of a mutation in the sequence due to deletion, insertion, non-conservative or conservative substitution of the amino acid sequence of the polypeptide or the polynucleotide sequence encoding the polypeptide, or a combination thereof; or replacement with an amino acid sequence or polynucleotide sequence modified to exhibit stronger activity or an amino acid sequence or polynucleotide sequence modified to have increased activity so that the activity of the polypeptide is enhanced, but is not limited thereto. The replacement may be specifically performed by inserting a polynucleotide into the chromosome by homologous recombination, but is not limited thereto. The vector used herein may further include a selection marker for the confirmation of chromosome insertion. The selection marker is as described above.
6) The introduction of a foreign polynucleotide exhibiting the activity of the polypeptide may be introduction of a foreign polynucleotide encoding a polypeptide exhibiting activity identical/similar to that of the polypeptide into a host cell. There is no limitation on its origin or sequence as long as the foreign polynucleotide exhibits activity identical/similar to that of the polypeptide. The method used in the introduction may be performed by appropriately selecting a known transformation method by those skilled in the art. As the introduced polynucleotide is expressed in a host cell, the polypeptide may be produced, and the activity thereof may be increased.
7) The codon optimization of the polynucleotide encoding the polypeptide may be codon optimization of an endogenous polynucleotide so as to increase the transcription or translation of the polynucleotide in a host cell, or codon optimization of a foreign polynucleotide so as to perform optimized transcription and translation of the polynucleotide in a host cell.
8) The analysis of the tertiary structure of the polypeptide to select and modify or chemically modify the exposed site may be, for example, to determine a template protein candidate according to the degree of similarity of the sequence by comparing the sequence information of a polypeptide to be analyzed with a database storing the sequence information of known proteins, to confirm the structure based on the same, and to select and modify or chemically modify the exposed site to be modified or chemically modified.

Such enhancement of polypeptide activity may be an increase in the activity or concentration or expression level of the corresponding polypeptide, based on the activity or concentration of the polypeptide expressed in a wild-type or a microbial strain before modification, or an increase in the amount of a product produced from the corresponding polypeptide, but is not limited thereto.

In the microorganism of the present disclosure, modification of all or part of the polynucleotide may be induced by (a) homologous recombination using a vector for chromosome insertion in the microorganism or genome editing using engineered nuclease (*e.g.*, CRISPR-Cas9) and/or (b) treatment with light (*e.g.*, UV rays, radiation, *etc.),* and/or chemicals, but is not limited thereto. A method of modifying all or part of the gene may include a method using a DNA recombination technology. For example, all or part of the gene may be deleted by introducing a nucleotide sequence or vector including a nucleotide sequence homologous to the target gene into the microorganism to cause homologous recombination. The nucleotide sequence or vector to be introduced may include a dominant selection marker, but is not limited thereto.

In the microorganism of the present disclosure, the variant, polynucleotide, retinoid, *etc.* are as described in the other aspects above.

Still another aspect of the present disclosure provides a method for producing a retinoid, which includes culturing a microorganism including the variant of the present disclosure or the polynucleotide of the present disclosure in a medium.

As used herein the term "culture" refers to growing the microorganism of the present disclosure in appropriately adjusted environmental conditions. The culture procedure of the present disclosure may be performed according to appropriate media or culture conditions known in the art. Such culture may easily be adjusted according to the selected microorganism by those skilled in the art. Specifically, the culture may be in a batch type, a continuous type, and/or a fed-batch type, but is not limited thereto.

As used herein, the "medium" refers to a mixture of materials containing, as main ingredients, nutrient materials required for culturing the microorganism of the present disclosure, in which the medium supplies nutrient materials including water essential for survival and growth, growth factors, *etc.* Specifically, as the media and other culture conditions used for culturing the microorganism of the present disclosure, any medium used for general culture of microorganisms may be used without particular limitation; however, the microorganism of the present disclosure may be cultured under aerobic conditions in a common medium containing appropriate carbon sources, nitrogen sources, phosphorus sources, inorganic compounds, amino acids, and/or vitamins, *etc.* while controlling the temperature, pH, *etc.*

In the present disclosure, the carbon source may include carbohydrates (*e.g.*, glucose, saccharose, lactose, fructose, sucrose, maltose, *etc.);* sugar alcohols (*e.g.*, mannitol, sorbitol, *etc.*); organic acids (*e.g.*, pyruvic acid, lactic acid, citric acid, *etc.);* and amino acids (*e.g.*, glutamic acid, methionine, lysine, *etc.).* Additionally, natural organic nutrient sources (*e.g.*, starch hydrolysates, molasses, blackstrap molasses, rice bran, cassava, bagasse, and corn steep liquor) may be used, and specifically, carbohydrates (*e.g.*, glucose and sterilized pretreated molasses (*i.e.*, molasses converted to reducing sugars)) may be used, and appropriate amounts of various other carbon sources may be variously used without limitation. These carbon sources may be used alone or in a combination of two or more thereof, but are not limited thereto.

As the nitrogen sources, inorganic nitrogen sources (*e.g.*, ammonia, ammonium sulfate, ammonium chloride, ammonium acetate, ammonium phosphate, ammonium carbonate, ammonium nitrate, *etc.*); amino acids (*e.g.*, glutamic acid, methionine, glutamine, *etc.);* and organic nitrogen sources (*e.g.*, peptone, NZ-amine, meat extracts, yeast extracts, malt extracts, corn steep liquor, casein hydrolysates, fishes or decomposition products thereof, defatted soybean cake or degradation products thereof, *etc.)* may be used. These nitrogen sources may be used alone or in a combination of two or more thereof, but are not limited thereto.

The phosphate sources may include potassium dihydrogen phosphate, dipotassium hydrogen phosphate, and sodium-containing salts corresponding thereto. As inorganic compounds, sodium chloride, calcium chloride, iron chloride, magnesium sulfate, iron sulfate, manganese sulfate, calcium carbonate, *etc.* may be used, and in addition, amino acids, vitamins, and/or suitable precursors may be included. These components or precursors may be added to the medium in a batch or continuous manner. However, the present disclosure is not limited thereto.

Additionally, the pH of the medium may be adjusted by adding compounds, such as ammonium hydroxide, potassium hydroxide, ammonia, phosphoric acid, and sulfuric acid, to the medium in an appropriate manner during the culture of the microorganism of the present disclosure. Additionally, an anti-foaming agent (*e.g.*, a fatty acid polyglycol ester) may be added to inhibit foam formation during the culture. Additionally, oxygen or oxygen-containing gas may be injected into the medium to maintain the aerobic state of the medium, or no gas may be injected, or nitrogen, hydrogen or carbon dioxide gas may be injected into the medium to maintain the anaerobic or microaerobic state of the medium, but the present disclosure is not limited thereto.

In the culture of the present disclosure, the culture temperature may be maintained at 20°C to 45°C, specifically at 25°C to 40°C, 20°C to 35°C, or 25°C to 35°C, and the culture may be performed for about 10 hours to about 160 hours, but is not limited thereto.

The retinoid produced by the culture of the present disclosure may be released into the medium or may remain in the cells.

The method of producing a retinoid of the present disclosure may further include preparing the microorganism of the present disclosure, preparing a medium for culturing the microorganism, or a combination thereof (in any order), for example, before or after the culture.

The method of producing a retinoid of the present disclosure may further include a step of recovering a retinoid from a medium resulting from the culture (a medium in which culture has been performed) or from the microorganism of the present disclosure. The recovery step may further be included after the culturing step.

The recovery may be collecting the desired retinoid using an appropriate method known in the art according to the method of culturing the microorganism of the present disclosure, for example, a batch, continuous, or fed-batch type culture. For example, the recovery may be performed using centrifugation, filtration, treatment with a crystallized protein precipitating agent (salting-out), extraction, cell disruption, sonication, ultrafiltration, dialysis, various types of chromatography (e.g., molecular sieve chromatography (gel filtration), adsorption chromatography, ion exchange chromatography, affinity chromatography, *etc.),* HPLC, and a combination of these methods, and the desired retinoid may be recovered from the medium or microorganism using an appropriate method known in the art.

Additionally, the method of producing a retinoid of the present disclosure may further include a step of purification. The purification may be performed using an appropriate method known in the art. In an embodiment, when the method of producing a retinoid of the present disclosure includes both the recovery step and the purification step, the recovery step and the purification step may be performed continuously or discontinuously regardless of the order, or may be performed simultaneously or as a single integrated step, but the method is not limited thereto.

In the present disclosure, in the method of producing a retinol derivative, which is a type of retinoid, the culturing step may further include a step of converting retinol into a retinol derivative. In the method of producing a retinol derivative of the present disclosure, the conversion step may further be included after the culturing step or the recovery step. The conversion step may be performed using an appropriate method known in the art (Jang et al. Microbial Cell Factories 2011, 10:59, Choi et al. Antioxidants. 2020).

In the method of the present disclosure, the variant, polynucleotide, vector, microorganism, *etc.* are as described in the other embodiments above.

Still another aspect of the present disclosure provides a composition for producing a retinoid, which includes: a microorganism including the variant of the present disclosure, the polynucleotide encoding the variant, the vector including the polynucleotide, or the polynucleotide of the present disclosure; a medium in which the microorganism was cultured; or a combination of two or more thereof.

The composition of the present disclosure may further include any appropriate excipient commonly used in the composition for producing a retinoid, and examples of the excipient may include a preservative, a wetting agent, a dispersing agent, a suspending agent, a buffer, a stabilizing agent, an isotonic agent, *etc.,* but the excipient is not limited thereto.

In the composition of the present disclosure, the variant, polynucleotide, vector, microorganism, medium, retinoid, *etc.* are as described in the other embodiments above.

Still another aspect of the present disclosure provides use of any one or more of the variant, polynucleotide, vector, and microorganism of the present disclosure, for producing a retinoid.

The variant, polynucleotide, vector, microorganism, retinoid, *etc.* are as described in the other embodiments above.

### [Mode for Carrying Out the Invention]

Hereinafter, the present disclosure will be described in more detail with reference to Examples. However, these Examples are only for illustrating the present disclosure, and the scope of the present disclosure is not intended to be limited by these Examples. Meanwhile, technical matters not described in this specification can sufficiently be understood and easily be practiced by those skilled in the art of the present disclosure or similar technical fields.

### Example 1. Preparation of BCO variant vector library

In order to amplify the BCO gene derived from the uncultured marine bacterium 66A03, the amino acid sequence of SEQ ID NO: 1 and the polynucleotide sequences of SEQ ID NO: 2 were obtained based on the amino acid sequence (Q4PNI0) registered in the UniProtKB (UniProt Knowledgebase). A Diversify PCR Random Mutagenesis Kit (Takara) was used to induce random mutations through error-prone PCR. PCR conditions and methods were optimized according to Buffer Condition 1 and Buffer Condition 2 in the user manual, respectively. Specific error-prone PCR conditions are shown in Table 1 below.

PCR was performed using the wild-type BCO gene (SEQ ID NO: 2) derived from the marine bacterium 66A03, which was synthesized by Macrogen Inc. by request, as a template, and the primers of SEQ ID NO: 5 and SEQ ID NO: 6. PCR conditions were as follows: 25 cycles of denaturation at 95°C for 30 seconds; annealing at 55°C for 30 seconds; and polymerization at 68°C for 1 minute. The BCO products obtained by PCR amplification under the two different conditions were combined to prepare a library of BCO variant vectors based on the pIMR53-TEFINtp-CYC1t vector.

**[Table 1]**

| | Buffer Condition 1 | Buffer Condition 2 |
|---|---|---|
| PCR-grade water | 40 µL | 39 µL |
| 10X Titanium Taq buffer | 5 µL | 5 µL |
| MnSO₄ | 0 µL | 1 µL |
| dGTP | 1 µL | 1 µL |
| 50X Diversify dNTP Mix | 1 µL | 1 µL |
| Primer mix | 1 µL | 1 µL |
| Template DNA | 1 µL | 1 µL |
| Titanium Taq polymerase | 1 µL | 1 µL |

The pIMR53-TEFINtp-CYC1t vector was prepared as follows.

In order to amplify TEFINtp, PCR was performed using *Yarrowia lipolytica* PO1f genomic DNA as a template and primers of SEQ ID NO: 7 and SEQ ID NO: 8; and in order to amplify the CYC1 terminator, PCR was performed using *Saccharomyces cerevisiae* genomic DNA as a template and primers of SEQ ID NO: 9 and SEQ ID NO: 10. PCR conditions were as follows: 32 cycles of denaturation at 95°C for 1 minute; annealing at 55°C for 1 minute; and polymerization at 68°C for 1 minute. Additionally, PCR was performed using the pIMR53 vector as a template and primers of SEQ ID NO: 11 and SEQ ID NO: 12. PCR conditions were as follows: 32 cycles of denaturation at 95°C for 1 minute; annealing at 55°C for 1 minute; and polymerization at 68°C for 5 minutes.

As a result, 6,774 bp of linearized pIMR53, 531 bp of TEFINtp, and 248 bp of CYC1t were obtained. The DNA fragments of TEFINtp and CYC1t obtained by PCR amplification were ligated into the pIMR53 vector using the Infusion cloning kit (Invitrogen), transformed into *E. coli* DH5α, and the transformants were plated on LB solid medium containing 30 mg/L ampicillin.

The colonies transformed with the plasmid, into which the desired TEFINt promoter and CYC1 terminator were inserted, were selected through PCR, and then the plasmid was obtained using a commonly known plasmid extraction method, and the plasmid was named pIMR53-TEFINtp-CYC1t.

### Example 2. Preparation Yarrowia lipolytica platform strain for retinoid production

### Example 2-1. Preparation of strain into which crtYB-crtl derived from X. dendrorhous is inserted

In order to prepare a platform strain for producing retinoids, the genes for lycopene cyclase/phytoene synthase (crtYB) and phytoene desaturase (crtl) genes derived from *Xanthophyllomyces dendrorhous* were inserted into the genome of *Yarrowia lipolytica* CC08-0125 strain (Accession No. KCCM12972P), which is a high-fat yeast. As for crtYB, the polynucleotide of SEQ ID NO: 13 was obtained based on the nucleotide sequence (GenBank: AY177204.1) registered in the National Center for the Biotechnology Information Search database (NCBI), and as for crtl, the polynucleotide of SEQ ID NO: 14 was obtained based on the nucleotide sequence (GenBank: Based on AY177424.1) registered in the NCBI. The polynucleotide sequences of crtYB and crtl were synthesized by Macrogen Inc. in the form of TEFINtp-crtYB-CYC1t (SEQ ID NO: 15) and TEFINtp-crtl-CYC1t (SEQ ID NO: 16), and a cassette to be inserted into the locus of the MHY1 (YALI0B21582g) gene was designed using URA3 gene of Y. *lipolytica* (SEQ ID NO: 17) as a selection marker. Each PCR was performed using the synthesized *crtYB* and *crtI* genes and KCCM12972P genomic DNA as templates and the primer pairs of SEQ ID NO: 18 and SEQ ID NO: 19, SEQ ID NO: 20 and SEQ ID NO: 21, SEQ ID NO: 22 and SEQ ID NO: 23, SEQ ID NO: 24 and SEQ ID NO: 25, SEQ ID NO: 26 and SEQ ID NO: 27, and SEQ ID NO: 28 and SEQ ID NO: 29, respectively. PCR conditions were as follows: 35 cycles of denaturation at 95°C for 1 minute; annealing at 55°C for 1 minute; and polymerization at 72°C for 3 minutes and 30 seconds. The thus-obtained five DNA fragments were prepared as a single cassette through overlap extension PCR.

The thus-prepared cassette was introduced into the KCCM12972P strain by a heat shock method (D.-C. Chen et al., Appl Microbiol Biotechnol, 1997), and then colonies formed on a uracil-free solid medium (YLMM1) were obtained. The colonies, in which the insertion of the cassette into the genome was confirmed using the primers of SEQ ID NO: 30 and SEQ ID NO: 31, were spotted on a 5-FOA solid medium and cultured at 30°C for 3 days. The URA3 marker was recovered by obtaining the colonies grown on the 5-FOA solid medium.

### Example 2-2. Preparation of HMGR-enhanced strain

A cassette was designed to replace, with a TEFINt promoter, a native promoter (SEQ ID NO: 32) region of 3-hydroxy-3-methylglutaryl-CoA reductase (HMGR) gene of the strain prepared in Example 2-1, into which the *crtYB* and *crtI* genes were inserted. Each PCR was performed using KCCM12972P genomic DNA as a template, and the primer pairs of SEQ ID NO: 33 and SEQ ID NO: 34, SEQ ID NO: 35 and SEQ ID NO: 36, SEQ ID NO: 37 and SEQ ID NO: 38, SEQ ID NO: 39 and SEQ ID NO: 40, and SEQ ID NO: 41 and SEQ ID NO: 42, respectively. PCR conditions were as follows: 35 cycles consisting of denaturation at 95°C for 1 minute; annealing at 55°C for 1 minute; and polymerization at 72°C for 1 minute and 30 seconds. The thus-obtained five DNA fragments were prepared as a single cassette through overlap extension PCR.

The thus-prepared cassette was introduced into the strain prepared in Example 2-1 by a heat shock method, and then colonies formed on a solid medium (YLMM1) without uracil were obtained. The colonies, in which the insertion of the cassette was confirmed using the primers of SEQ ID NO: 43 and SEQ ID NO: 44, were spotted on a 5-FOA solid medium and cultured at 30°C for 3 days. The URA3 marker was recovered by obtaining the colonies grown on the 5-FOA solid medium. The retinoid-producing platform strain ultimately prepared was named CC08-1023.

### <Yarrowia lipolytica Minimal Media 1 (YLMM1)>

20 g/L of glucose, 6.7 g/L of yeast nitrogen base without amino acids, 2 g/L of yeast synthetic drop-out medium supplements without uracil, and 15 g/L of agar

### <5-Fluoroorotic Acid (5-FOA)>

20 g/L of glucose, 6.7 g/L of yeast nitrogen base without amino acids, 2 g/L of yeast synthetic drop-out medium supplements without uracil, 50 µg/mL of uracil, 1 g/L of 5-fluoroorotic acid (5-FOA), and 15 g/L of agar

### Example 3. Primary selection of BCO activity-enhanced strain

For selection of BCO activity-enhanced strains, pIMR53 (negative control; NC), pIMR53-TEFINtp-BCO (wild-type, SEQ ID NO: 2)-CYC1t vector (positive control; PC), and the BCO random variant-expressing vector library prepared in Example 1 were each transformed into the CC08-1023 strain, which is a platform strain for producing retinoids, by heat shock. Thereafter, the colonies formed on a solid medium without uracil were obtained.

350 µL of a liquid medium of *Yarrowia lipolytica* minimal media 2 (YLMM2) without uracil was dispensed into each well of 96-deep-well plates (31 plates), and the thus-obtained colonies were inoculated and cultured at 30°C for 48 hours. Thereafter, the colonies were spotted on a YLMM1 solid medium without uracil, and cultured at 30°C for 3 days. After completion of the culture, 17 kinds of strains, which were lighter in color compared to the strain transformed with the positive control (CC08-1023/pIMR53-TEFINtp-BCO-CYC1t), were first selected from a total of 3,000 strains. The method of preparing the above described YLMM2 and the composition thereof are as follows.

### <YLMM2 (pH 7.0)>

40 g/L of glucose, 1.7 g/L of yeast nitrogen base without amino acids and ammonium sulfate, 0.5 g/L of uracil, and 2.5 g/L of ammonium sulfate dissolved in 0.1 M sodium phosphate buffer (pH 7.0)

### Example 4. Secondary selection of BCO activity-enhanced strain

A flask test was performed on the 17 strains obtained by primary selection in Example 3.

In a 250 mL corner-baffled flask containing 50 mL of *Yarrowia lipolytica* minimal media 2 (YLMM2) without uracil, the 17 kinds of strains and the strain transformed with the positive control (PC) vector were each inoculated at an initial OD of 1 and cultured at 30°C for 48 hours with shaking at 250 rpm. After completion of the culture, 1 mL of the culture medium was centrifuged and the supernatant was discarded. Then, 0.5 mL of dimethyl sulfoxide (DMSO, Sigma) was added thereto, and the cells were disrupted by shaking at 2,000 rpm at 55°C for 10 minutes. Additionally, 0.5 mL of acetone (Sigma) was added and shaken at 2,000 rpm at 45°C for 15 minutes to extract retinol and retinal, and the concentrations of the extracted materials were analyzed using HPLC equipment. The results of the flask test on the selected strains, and the results of measuring OD, concentrations, and contents of retinol and retinal are shown in Table 2 below.

**[Table 2]**

| Number | OD (A600) | Retinol (mg/L) | Retinal (mg/L) | Retinol (%) |
|---|---|---|---|---|
| NC | 65 | ND | ND | - |
| PC | 61 | 8.2 | 1.4 | 100 |
| 1 | 64 | 12.0 | 1.9 | 146 |
| 2 | 42 | 7.0 | 1.2 | 85 |
| 3 | 32 | 4.9 | 0.8 | 59 |
| 4 | 66 | 9.7 | 1.5 | 118 |
| 5 | 57 | 9.3 | 1.5 | 113 |
| 6 | 44 | 6.1 | 1.0 | 74 |
| 7 | 47 | 6.0 | 1.0 | 72 |
| 8 | 38 | 5.9 | 1.0 | 72 |
| 9 | 56 | 7.1 | 1.2 | 86 |
| 10 | 47 | 7.0 | 1.1 | 85 |
| 11 | 58 | 7.7 | 1.3 | 94 |
| 12 | 52 | 8.3 | 1.3 | 101 |
| 13 | 46 | 7.1 | 1.2 | 86 |
| 14 | 46 | 7.1 | 1.2 | 86 |
| 15 | 54 | 6.9 | 1.1 | 84 |
| 16 | 58 | 8.7 | 1.4 | 106 |
| 17 | 54 | 7.7 | 1.3 | 94 |

Thereafter, the concentrations of retinol and retinal extracted from the test samples were used as a standard for secondary selection. It was confirmed that among the strains evaluated, five strains (Nos. 1, 4, 5, 12, and 16) showed an increase in retinol concentration (Retinol (%)) compared to the positive control (PC). In particular, it was confirmed that in the case of strain No. 1, the retinol concentration was increased by about 46% compared to that of the positive control (PC). Therefore, the plasmid transformed into the strain No. 1, which showed the highest increase in retinol concentration, was extracted and sequence analysis was performed to identify random mutations located in the BCO ORF. The transformed BCO plasmid was extracted from the selected strain using the Zymoprep Yeast Plasmid Miniprep Kit 2 (Zymo Research), and sequence analysis was performed in both directions using SEQ ID NO: 45 and SEQ ID NO: 46 to increase the reliability of sequencing. As a result, the number of mutations in the BCO ORF was confirmed to be three, and the confirmed mutations are shown in Table 3.

**[Table 3]**

| Number of Variant Strain | Amino Acid Seq. (Nucleotide Seq.) |
|---|---|
| 1 | A43V (GCC→GTC) |
| | L58Q (CTG→CAG) |
| | I74V (ATC→GTC) |

From the results above, it was confirmed that the introduction of the BCO variant of the present disclosure can increase the amount of retinoid production.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds, are therefore intended to be embraced by the claims.

### Sequence Listing

Sequences of the present disclosure are shown in Tables 4 to 6 below.

**[Table 4]**

| SEQ ID NO: | Name | Sequence | Type |
|---|---|---|---|
| SEQ ID NO: 1 | Blh_ WT | | protein |
| SEQ ID NO: 2 | blh_W T | atgggcttga tgttaattga ttggtgtgct ttagcattgg ttgtgtttat tggtttgcca 60catggtgcct tagatgctgc tatttctttt tcaatgattt cttcagcaaa gagaattgct 120 agattagcag gaatactatt aatttacctg ttgttagcaa ccgcattttt tttaatttgg 180 tatcaattac cagcattttc tcttcttatt tttcttttga taagcataat ccattttgga 240 atggctgatt tcaatgcatc cccaagtaaa cttaagtggc ctcatattat tgcacatggc 300 ggcgttgtta ctgtttggtt gccgcttatc caaaaaaatg aagttacgaa gctattttca 360 atattaacaa atggtccaac tcccatttta tgggacatac | DNA |
| | | tattgatatt tttttatgt 420 tggagcatag gagtatgtct tcatacctat gaaactttac gttctaaaca ttataatatc 480 gcctttgaac ttattggatt aatttttcta gcctggtatg cacccccact cgttactttt 540 gccacatact tctgctttat ccacagcaga cgtcacttta gttttgtttg gaaacagtta 600 cagcatatga gttcaaaaaa aatgatgata ggtagtgcca ttattttatc ttgtacgagc 660 tggttgatag gcggaggaat atattttttc ctcaattcga aaatgattgc cagtgaagct 720 gctttacaaa ctgtctttat tggtcttgca gctttaacag ttcctcacat gatacttatc 780 gactttatat ttagaccaca ctcttccaga attaaaatca aaaattga 828 | |
| SEQ ID NO: 3 | Blh_V T | MGLMLIDWCA LALVVFIGLP HGALDAAISF SMISSAKRIA RLVGILLIYL LLATAFFQIW 60YQLPAFSLLI FLLVSIIHFG MADFNASPSK LKWPHIIAHG GVVTVWLPLI QKNEVTKLFS          120 ILTNGPTPIL WDILLIFFLC WSIGVCLHTY ETLRSKHYNI AFELIGLIFL AWYAPPLVTF 180 ATYFCFIHSR RHFSFVWKQL QHMSSKKMMI GSAIILSCTS WLIGGGIYFF LNSKMIASEA          240 ALQTVFIGLA ALTVPHMILI DFIFRPHSSR IKIKN | protein |
| SEQ ID NO: 4 | blh_V T | atgggcctga tgctgatcga ctggtgtgcc ctggccctgg tggtgttcat cggcctgccc 60cacggcgccc tggacgccgc catctctttc tctatgatct cttctgccaa gcgaatcgcc | DNA |
| | | 120 cgactggtcg gcatcctgct gatctacctg ctgctggcca ccgccttctt ccagatctgg 180 taccagctgc ccgccttctc tctgctgatc ttcctgctgg tctctatcat ccacttcggc 240 atggccgact tcaacgcctc tccctctaag ctgaagtggc cccacatcat cgcccacggc 300 ggcgtggtga ccgtgtggct gcccctgatc cagaagaacg aggtgaccaa gctgttctct 360 atcctgacca acggccccac ccccatcctg tgggacatcc tgctgatctt cttcctgtgt 420 tggtctatcg gcgtgtgtct gcacacctac gagaccctgc gatctaagca ctacaacatc 480 gccttcgagc tgatcggcct gatcttcctg gcctggtacg cccccccct ggtgaccttc 540 gccacctact tctgtttcat ccactctcga cgacacttct ctttcgtgtg gaagcagctg 600 cagcacatgt cttctaagaa gatgatgatc ggctctgcca tcatcctgtc ttgtacctct 660 tggctgatcg gcggcggcat ctacttcttc ctgaactcta agatgatcgc ctctgaggcc 720 gccctgcaga ccgtgttcat cggcctggcc gccctgaccg tgccccacat gatcctgatc 780 gacttcatct tccgacccca ctcttctcga atcaagatca agaactag 828 | |
| SEQ ID NO: 5 | BCO-F | ttttgcagta ctaaccgcag ggcctgatgc tgatcgactg | DNA |
| SEQ ID NO: 6 | BCO-R | tgacataact aattacatga ctagttcttg atcttgattc | DNA |
| SEQ ID NO: 7 | TEFI Ntp-F | cctttcgtct tcaaggaatt agagaccggg ttggcggcgc | DNA |
| SEQ ID NO: 8 | TEFI Ntp-R | cgtgacataa ctaattacat gactgcggtt agtactgcaa aaag | DNA |
| SEQ ID NO: 9 | CYC1 t-F | ctttttgcag tactaaccgc agtcatgtaa ttagttatgt cacg | DNA |
| SEQ ID NO: 10 | CYC1 t-R | tcgtcgcggc cgcggcgcgc cggcaaatta aagccttcga gcgt | DNA |
| SEQ ID NO: 11 | pIMR 53-F | gcgccgccaa cccggtctct aattccttga agacgaaagg | DNA |
| SEQ ID NO: 12 | pIMR 53-R | acgctcgaag gctttaattt gccggcgcgc cgcggccgcg acga | DNA |
| SEQ ID NO: 13 | X. dendr orhou s crtYB | atgacggctc tcgcatatta ccagatccat ctgatctata ctctcccaat tcttggtctt 60ctcggcctgc tcacttcccc gattttgaca aaatttgaca tctacaaaat atcgatcctc 120 gtatttattg cgtttagtgc aaccacacca tgggactcat ggatcatcag aaatggcgca 180 tggacatatc catcagcgga gagtggccaa ggcgtgtttg gaacgtttct agatgttcca 240 tatgaagagt acgctttctt tgtcattcaa accgtaatca ccggcttggt ctacgtcttg 300 gcaactaggc accttctccc atctctcgcg cttcccaaga ctagatcgtc cgccctttct 360 ctcgcgctca aggcgctcat ccctctgccc attatctacc tatttaccgc tcaccccagc 420 ccatcgcccg acccgctcgt gacagatcac tacttctaca tgcgggcact ctccttactc 480 atcaccccac ctaccatgct cttggcagca ttatcaggcg aatatgcttt cgattggaaa 540 agtggccgag caaagtcaac tattgcagca atcatgatcc cgacggtgta tctgatttgg 600 gtagattatg ttgctgtcgg tcaagactct tggtcgatca | DNA |
| | | acgatgagaa gattgtaggg 660 tggaggcttg gaggtgtact acccattgag gaagctatgt tcttcttact gacgaatcta 720 atgattgttc tgggtctgtc tgcctgcgat catactcagg ccctatacct gctacacggt 780 cgaactattt atggcaacaa aaagatgcca tcttcatttc ccctcattac accgcctgtg 840 ctctccctgt tttttagcag ccgaccatac tcttctcagc caaaacgtga cttggaactg 900 gcagtcaagt tgttggagga aaagagccgg agcttttttg ttgcctcggc tggatttcct 960 agcgaagtta gggagaggct ggttggacta tacgcattct gccgggtgac tgatgatctt 1020 | |
| | | atcgactctc ctgaagtatc ttccaacccg catgccacaa ttgacatggt ctccgatttt          1080 cttaccctac tatttgggcc cccgctacac ccttcgcaac ctgacaagat cctttcttcg          1140 cctttacttc ctccttcgca cccttcccga cccacgggaa tgtatcccct cccgcctcct          1200 ccttcgctct cgcctgccga gctcgttcaa ttccttaccg aaagggttcc cgttcaatac          1260 catttcgcct tcaggttgct cgctaagttg caagggctga tccctcgata cccactcgac          1320 gaactcctta gaggatacac cactgatctt atctttccct tatcgacaga ggcagtccag          1380 gctcggaaga cgcctatcga gaccacagct gacttgctgg actatggtct atgtgtagca 1440 ggctcagtcg ccgagctatt ggtctatgtc tcttgggcaa gtgcaccaag tcaggtccct          1500 gccaccatag aagaaagaga agctgtgtta gtggcaagcc gagagatggg aactgccctt | |
| | | 1560 cagttggtga acattgctag ggacattaaa ggggacgcaa cagaagggag attttaccta 1620 ccactctcat tctttggtct tcgggatgaa tcaaagcttg cgatcccgac tgattggacg 1680 gaacctcggc ctcaagattt cgacaaactc ctcagtctat ctccttcgtc cacattacca 1740 tcttcaaacg cctcagaaag cttccggttc gaatggaaga cgtactcgct tccattagtc 1800 gcctacgcag aggatcttgc caaacattct tataagggaa ttgaccgact tcctaccgag 1860 gttcaagcgg gaatgcgagc ggcttgcgcg agctacctac tgatcggccg agagatcaaa 1920 gtcgtttgga aaggagacgt cggagagaga aggacagttg ccggatggag gagagtacgg 1980 aaagtcttga gtgtggtcat gagcggatgg gaagggcag 2019 | |
| SEQ ID NO: 14 | X. dendr orhou s crtl | atgggaaaag aacaagatca ggataaaccc acagctatca tcgtgggatg tggtatcggt 60ggaatcgcca ctgccgctcg tcttgctaaa gaaggtttcc aggtcacggt gttcgagaag 120 aacgactact ccggaggtcg atgctcttta atcgagcgag atggttatcg attcgatcag 180 gggcccagtt tgctgctctt gccagatctc ttcaagcaga cattcgaaga tttgggagag          240 | DNA |
| | | aagatggaag attgggtcga tctcatcaag tgtgaaccca actatgtttg ccacttccac 300 gatgaagaga ctttcactct ttcaaccgac atggcgttgc tcaagcggga agtcgagcgt 360 tttgaaggca aagatggatt tgatcggttc ttgtcgttta tccaagaagc ccacagacat 420 tacgagcttg ctgtcgttca cgtcctgcag aagaacttcc ctggcttcgc agcattctta 480 cggctacagt tcattggcca aatcctggct cttcacccct tcgagtctat ctggacaaga 540 gtttgtcgat atttcaagac cgacagatta cgaagagtct tctcgtttgc agtgatgtac 600 atgggtcaaa gcccatacag tgcgcccgga acatattcct tgctccaata caccgaattg 660 accgagggca tctggtatcc gagaggaggc ttttggcagg ttcctaatac tcttcttcag 720 atcgtcaagc gcaacaatcc ctcagccaag ttcaatttca acgctccagt ttcccaggtt 780 cttctctctc ctgccaagga ccgagcgact ggtgttcgac ttgaatccgg cgaggaacat 840 cacgccgatg ttgtgattgt caatgctgac ctcgtttacg cctccgagca cttgattcct 900 gacgatgcca gaaacaagat tggccaactg ggtgaagtca agagaagttg gtgggctgac 960 ttagttggtg gaaagaagct caagggaagt tgcagtagtt tgagcttcta ctggagcatg 1020 | |
| | | gaccgaatcg tggacggtct gggcggacac aatatcttct tggccgagga cttcaaggga | |
| | | 1080 tcattcgaca caatcttcga ggagttgggt ctcccagccg atccttcctt ttacgtgaac 1140 gttccctcgc gaatcgatcc ttctgccgct cccgaaggca aagatgctat cgtcattctt 1200 gtgccgtgtg gccatatcga cgcttcgaac cctcaagatt acaacaagct tgttgctcgg 1260 gcaaggaagt ttgtgatcca cacgctttcc gccaagcttg gacttcccga ctttgaaaaa 1320 atgattgtgg cagagaaggt tcacgatgct ccctcttggg agaaagaatt caacctcaag 1380 gacggaagca tcttgggact ggctcacaac tttatgcaag ttcttggttt caggccgagc 1440 accagacatc ccaagtatga caagttgttc tttgtcgggg cttcgactca tcccggaact 1500 ggggttccca tcgtcttggc tggagccaag ttaactgcca accaagttct cgaatccttt 1560 gaccgatccc cagctccaga tcccaatatg tcactctccg taccatatgg aaaacctctc 1620 aaatcaaatg gaacgggtat cgattctcag gtccagctga agttcatgga tttggagaga 1680 tgggtatacc ttttggtgtt gttgattggg gccgtgatcg ctcgatccgt tggtgttctt 1740 gctttctgaa gcaagacaac gatcctttct tagagtttta ttttagtctc ttcctgtgtt 1800 ctctctatat acatactctg ctcgtctgtt ctcttctcga gggttcctct ttactttgtg 1860 tcagagtcat acccggtctc tcaacgtccg tttgagggct agacaattgt tagtctcgaa 1920 atctccatca cctcaagtct gatgttcatc atcttttta | |
| | | ttcgtt 1966 | |
| SEQ ID NO: 15 | TEFI Ntp-cr tYB-C YC1t | agagaccggg ttggcggcgc atttgtgtcc caaaaaacag ccccaattgc cccaattgac 60cccaaattga cccagtagcg ggcccaaccc cggcgagagc ccccttctcc ccacatatca 120 aacctccccc ggttcccaca cttgccgtta agggcgtagg gtactgcagt ctggaatcta 180 cgcttgttca gactttgtac tagtttcttt gtctggccat ccgggtaacc catgccggac 240 gcaaaataga ctactgaaaa tttttttgct ttgtggttgg gactttagcc aagggtataa 300 aagaccaccg tccccgaatt acctttcctc ttcttttctc tctctccttg tcaactcaca 360 cccgaaatcg ttaagcattt ccttctgagt ataagaatca ttcaaaatgg tgagtttcag 420 aggcagcagc aattgccacg ggctttgagc acacggccgg gtgtggtccc attcccatcg 480 acacaagacg ccacgtcatc cgaccagcac tttttgcagt actaaccgca gacggctctc 540 gcatattacc agatccatct gatctatact ctcccaattc ttggtcttct cggtctgctc 600 acttccccga ttttgacaaa atttgacatc tacaaaatat cgatcctcgt atttattgcg 660 tttagtgcaa ccacaccatg ggactcatgg atcatcagaa atggcgcatg gacatatcca 720 tcagcggaga gtggccaagg cgtgtttgga acgtttctag atgttccata tgaagagtac | DNA |
| | | 780 gctttctttg tcattcaaac cgtaatcacc ggcttggtct acgtcttggc aactaggcac 840 cttctcccat ctctcgcgct tcccaagact agatcgtccg ccctttctct cgcgctcaag 900 gcgctcatcc ctctgcccat tatctaccta tttaccgctc accccagccc atcgcccgac 960 ccgctcgtga cagatcacta cttctacatg cgggcactct ccttactcat caccccacct 1020 | |
| | | accatgctct tggcagcatt atcaggcgaa tatgctttcg attggaaaag tggccgagca          1080 aagtcaacta ttgcagcaat catgatcccg acggtgtatc tgatttgggt agattatgtt          1140 gctgtcggtc aagactcttg gtcgatcaac gatgagaaga ttgtagggtg gaggcttgga 1200 ggtgtactac ccattgagga agctatgttc ttcttactga cgaatctaat gattgttctg          1260 ggtctgtctg cctgcgatca tactcaggcc ctatacctgc tacacggtcg aactatttat          1320 ggcaacaaaa agatgccatc ttcatttccc ctcattacac cgcctgtgct ctccctgttt          1380 tttagcagcc gaccatactc ttctcagcca aaacgtgact tggaactggc agtcaagttg          1440 ttggaggaaa agagccggag cttttttgtt gcctcggctg gatttcctag cgaagttagg          1500 gagaggctgg ttggactata cgcattctgc cgggtgactg atgatcttat cgactctcct          1560 gaagtatctt ccaacccgca tgccacaatt gacatggtct ccgattttct taccctacta          1620 tttgggcccc cgctacaccc ttcgcaacct gacaagatcc tttcttcgcc tttacttcct          1680 | |
| | | ccttcgcacc cttcccgacc cacgggaatg tatcccctcc cgcctcctcc ttcgctctcg 1740 cctgccgagc tcgttcaatt ccttaccgaa agggttcccg ttcaatacca tttcgccttc 1800 aggttgctcg ctaagttgca agggctgatc cctcgatacc cactcgacga actccttaga 1860 ggatacacca ctgatcttat ctttccttta tcgacagagg cagtccaggc tcggaagacg 1920 cctatcgaga ccacagctga cttgctggac tatggtctat gtgtagcagg ctcagtcgcc 1980 gagctattgg tctatgtctc ttgggcaagt gcaccaagtc aggtccctgc caccatagaa 2040 gaaagagaag ctgtgttagt ggcaagccga gagatgggaa ctgcccttca gttggtgaac 2100 attgctaggg acattaaagg ggacgcaaca gaagggagat tttacctacc actctcattc 2160 tttggtcttc gggatgaatc aaagcttgcg atcccgactg attggacgga acctcggcct 2220 caagatttcg acaaactcct cagtctatct ccttcgtcca cattaccatc ttcaaacgcc 2280 tcagaaagct tccggttcga atggaagacg tactcgcttc cattagtcgc ctacgcagag 2340 gatcttgcca aacattctta taagggaatt gaccgacttc ctaccgaggt tcaagcggga 2400 atgcgagcgg cttgcgcgag ctacctactg atcggccgag agatcaaagt cgtttggaaa 2460 ggagacgtcg gagagagaag gacagttgcc ggatggagga gagtacggaa agtcttgagt 2520 | |
| | | gtggtcatga gcggatggga agggcagtaa ctcgagtcat gtaattagtt atgtcacgct 2580 tacattcacg ccctcccccc acatccgctc taaccgaaaa ggaaggagtt agacaacctg 2640 aagtctaggt ccctatttat ttttttatag ttatgttagt attaagaacg ttatttatat 2700 ttcaaatttt tctttttttt ctgtacagac gcgtgtacgc atgtaacatt atactgaaaa 2760 ccttgcttga gaaggttttg ggacgctcga aggctttaat ttgcggcgcg cggaggccct 2820 ttcgtcttca 2830 | |
| SEQ ID NO: 16 | TEFI Ntp-cr tl-CY C1t | agagaccggg ttggcggcgc atttgtgtcc caaaaaacag ccccaattgc cccaattgac 60cccaaattga cccagtagcg ggcccaaccc cggcgagagc ccccttctcc ccacatatca 120 aacctccccc ggttcccaca cttgccgtta agggcgtagg gtactgcagt ctggaatcta 180 cgcttgttca gactttgtac tagtttcttt gtctggccat ccgggtaacc catgccggac 240 gcaaaataga ctactgaaaa tttttttgct ttgtggttgg gactttagcc aagggtataa 300 aagaccaccg tccccgaatt acctttcctc ttcttttctc tctctccttg tcaactcaca 360 cccgaaatcg ttaagcattt ccttctgagt ataagaatca ttcaaaatgg tgagtttcag 420 aggcagcagc aattgccacg ggctttgagc acacggccgg gtgtggtccc attcccatcg 480 acacaagacg ccacgtcatc cgaccagcac | DNA |
| | | tttttgcagt actaaccgca gggaaaagaa 540 caagatcagg ataaacccac agctatcatc gtgggatgtg gtatcggtgg aatcgccact 600 gccgctcgtc ttgctaaaga aggtttccag gtcacggtgt tcgagaagaa cgactactcc 660 ggaggtcgat gctctttaat cgagcgagat ggttatcgat tcgatcaggg gcccagtttg 720 ctgctcttgc cagatctctt caagcagaca ttcgaagatt tgggagagaa gatggaagat 780 tgggtcgatc tcatcaagtg tgaacccaac tatgtttgcc acttccacga tgaagagact 840 ttcactcttt caaccgacat ggcgttgctc aagcgggaag tcgagcgttt tgaaggcaaa 900 gatggatttg atcggttctt gtcgtttatc caagaagccc acagacatta cgagcttgct 960 gtcgttcacg tcctgcagaa gaacttccct ggcttcgcag cattcttacg gctacagttc 1020 | |
| | | attggccaaa tcctggctct tcaccccttc gagtctatct ggacaagagt ttgtcgatat 1080 ttcaagaccg acagattacg aagagtcttc tcgtttgcag tgatgtacat gggtcaaagc 1140 ccatacagtg cgcccggaac atattccttg ctccaataca ccgaattgac cgagggcatc 1200 tggtatccga gaggaggctt ttggcaggtt cctaatactc ttcttcagat cgtcaagcgc 1260 aacaatccct cagccaagtt caatttcaac gctccagttt cccaggttct tctctctcct 1320 gccaaggacc gagcgactgg tgttcgactt gaatccggcg aggaacatca cgccgatgtt 1380 | |
| | | gtgattgtca atgctgacct cgtttacgcc tccgagcact tgattcctga cgatgccaga          1440 aacaagattg gccaactggg tgaagtcaag agaagttggt gggctgactt agttggtgga 1500 aagaagctca agggaagttg cagtagtttg agcttctact ggagcatgga ccgaatcgtg          1560 gacggtctgg gcggacacaa tatcttcttg gccgaggact tcaagggatc attcgacaca 1620 atcttcgagg agttgggtct cccagccgat ccttcctttt acgtgaacgt tccctcgcga          1680 atcgatcctt ctgccgctcc cgaaggcaaa gatgctatcg tcattcttgt gccgtgtggc          1740 catatcgacg cttcgaaccc tcaagattac aacaagcttg ttgctcgggc aaggaagttt 1800 gtgatccaca cgctttccgc caagcttgga cttcccgact ttgaaaaaat gattgtggca          1860 gagaaggttc acgatgctcc ctcttgggag aaagaattca acctcaagga cggaagcatc 1920 ttgggactgg ctcacaactt tatgcaagtt cttggtttca ggccgagcac cagacatccc          1980 aagtatgaca agttgttctt tgtcggggct tcgactcatc ccggaactgg ggttcccatc          2040 gtcttggctg gagccaagtt aactgccaac caagttctcg aatcctttga ccgatcccca          2100 gctccagatc ccaatatgtc actctccgta ccatatggaa aacctctcaa atcaaatgga          2160 acgggtatcg attctcaggt ccagctgaag ttcatggatt tggagagatg ggtatacctt          2220 | |
| | | ttggtattgt tgattggggc cgtgatcgct cgatccgttg gtgttcttgc tttctgactc          2280 gagtcatgta attagttatg tcacgcttac attcacgccc tccccccaca tccgctctaa          2340 ccgaaaagga aggagttaga caacctgaag tctaggtccc tatttatttt tttatagtta          2400 tgttagtatt aagaacgtta tttatatttc aaatttttct tttttttctg tacagacgcg          2460 tgtacgcatg taacattata ctgaaaacct tgcttgagaa ggttttggga cgctcgaagg          2520 ctttaatttgccggccgcga cgagtatctg tctga 2555 | |
| SEQ ID NO: 17 | Y. lipolyti ca URA3 | tgcctcctgt ctgactcgtc attgccgcct ttggagtacg actccaacta tgagtgtgct 60tggatcactt tgacgataca ttcttcgttg gaggctgtgg gtctgacagc tgcgttttcg 120 gcgcggttgg ccgacaacaa tatcagctgc aacgtcattg ctggctttca tcatgatcac 180 atttttgtcg gcaaaggcga cgcccagaga gccattgacg ttctttctaa tttggaccga 240 tagccgtata gtccagtcta tctataagtt caactaactc gtaactatta ccataacata 300 tacttcactg ccccagataa ggttccgata aaaagttctg cagactaaat ttatttcagt 360 ctcctcttca ccaccaaaat gccctcctac gaagctcgag ctaacgtcca caagtccgcc 420 tttgccgctc gagtgctcaa gctcgtggca gccaagaaaa ccaacctgtg tgcttctctg 480 gatgttacca ccaccaagga gctcattgag cttgccgata aggtcggacc ttatgtgtgc | DNA |
| | | 540 atgatcaaga cccatatcga catcattgac gacttcacct acgccggcac tgtgctcccc 600 ctcaaggaac ttgctcttaa gcacggtttc ttcctgttcg aggacagaaa gttcgcagat 660 attggcaaca ctgtcaagca ccagtacaag aacggtgtct accgaatcgc cgagtggtcc 720 gatatcacca acgcccacgg tgtacccgga accggaatca ttgctggcct gcgagctggt 780 gccgaggaaa ctgtctctga acagaagaag gaggacgtct ctgactacga gaactcccag 840 tacaaggagt tcctggtccc ctctcccaac gagaagctgg ccagaggtct gctcatgctg 900 gccgagctgt cttgcaaggg ctctctggcc actggcgagt actccaagca gaccattgag 960 cttgcccgat ccgaccccga gtttgtggtt ggcttcattg cccagaaccg acctaagggc 1020 gactctgagg actggcttat tctgaccccc ggggtgggtc ttgacgacaa gggagacgct 1080 ctcggacagc agtaccgaac tgttgaggat gtcatgtcta ccggaacgga tatcataatt 1140 gtcggccgag gtctgtacgg ccagaaccga gatcctattg aggaggccaa gcgataccag 1200 aaggctggct gggaggctta ccagaagatt aactgttaga ggttagacta tggatatgtc 1260 | |
| | | atttaactgt gtatatagag agcgtgcaag tatggagcgc ttgttcagct tgtatgatgg          1320 tcagacgacc tgtctgatcg agtatgtatg atactgcaca acctgtgtat ccgcatgatc          1380 tgtccaatgg ggcatgttgt tgtgtttctc gatacggaga tgctgggtac aagtagctaa          1440 tacgattgaa ctacttatac ttatatgagg cttgaagaaa gctgacttgt gtatgactta          1500 ttctcaactacatccccagtcacaatacca cca   1533 | |
| SEQ ID NO: 18 | Left arm1-F | gtgcgcttct ctcgtctcgg taaccctgtc | DNA |
| SEQ ID NO: 19 | Left arm1-R | atgcgccgcc aacccggtct ctggggtgtg gtggatgggg tgtg | DNA |
| SEQ ID NO: 20 | TEFI Ntp-cr tYB-C YC1t-F | cacaccccat ccaccacacc ccagagaccg ggttggcggc gcat | DNA |

**[Table 5]**

| | | | |
|---|---|---|---|
| SEQ ID NO: 21 | TEFI Ntp-cr tYB-C YC1t-R | cgccgccaac ccggtctctt gaagacgaaa gggcctccg | DNA |
| SEQ ID NO: 22 | TEFI Ntp-cr tl-CY C1t-F | cggaggccct ttcgtcttca agagaccggg ttggcggcg | DNA |
| SEQ ID NO: 23 | TEFI Ntp-cr tl-CY C1t-R | gacgagtcag acaggaggca tcagacagat actcgtcgcg | DNA |
| SEQ ID NO: 24 | Repe at-UR A3-F | cgcgacgagt atctgtctga tgcctcctgt ctgactcgtc | DNA |
| SEQ ID NO: 25 | Repe at-UR A3-R | atgacgagtc agacaggagg catggtggta ttgtgactgg ggat | DNA |
| SEQ ID NO: 26 | Repe at-F | atccccagtc acaataccac catgcctcct gtctgactcg tcat | DNA |
| SEQ ID NO: 27 | Repe at-R | cggcgtcctt ctcgtagtcc gcttttggtg gtgaagagga gact | DNA |
| SEQ ID NO: 28 | Right arm1-F | agtctcctct tcaccaccaa aagcggacta cgagaaggac gccg | DNA |
| SEQ ID NO: 29 | Right arm1-R | ccactcgtca ccaacagtgc cgtgtgttgc | DNA |
| SEQ ID NO: 30 | MHY1-CF | tcgtacgtct ataccaacag atgg | DNA |
| SEQ ID NO: 31 | MHY1 -CR | cgcatacaca cacactgccg gggg | DNA |
| SEQ ID NO: 32 | HMG R native promo ter region | tccacacgtc gttctttttt ccttagcctt ttttgcagtg cgcgtgtccc aaaccccagc 60tctacacacc agcacaaaca aagttaagct cagggttgtc gttgaggtcg cttactgtag 120 tcagtgctcg tatggttcgt tcaattttcg ccaaaaatcg ttttgccttt gtatcttggg 180 aataacatca actgtggttc ttcaacaggc ctaaggaacg aaacaagccg gaccaagatc 240 aggttcaagg tgagtactga gaaggaatag aaggcctaaa ggcgcaaacc gacaggtggc 300 aacagctcca caccgaccac gaaggccacg aaatcaaggg gtcctaaagt tagtctttgt 360 ggcctcgacg gtcagcgaaa acgcgagacc acaacgcgat cagaaccagg acctaaacaa 420 cacaggacgg ggtcacaata ggcttgaaca gcaagtacaa gctgtgatct ctctatattt 480 gattctcaaa ccacccctga ctacttcagc gcctctgtga cacagccccc ctatcatccg 540 actaacacag | DNA |
| SEQ ID NO: 33 | Left arm2-F | gacaatgcct cgaggaggtt taaaagtaac t | DNA |
| SEQ ID NO: 34 | Left arm2-R | gcgccgccaa cccggtctct ctgtgttagt cggatgatag g | DNA |
| SEQ ID NO: 35 | TEFI Ntp-F | cctatcatcc gactaacaca gagagaccgg gttggcggcg c | DNA |
| SEQ ID NO: 36 | TEFI Ntp-R | gacgagtcag acaggaggca ctgcggttag tactgcaaaa ag | DNA |
| SEQ ID NO: 37 | URA3 -F | ctttttgcag tactaaccgc agtgcctcct gtctgactcg tc | DNA |
| SEQ ID NO: 38 | URA3 -R | atgcgccgcc aacccggtct cttggtggta ttgtgactgg ggat | DNA |
| SEQ ID NO: 39 | Repe at-F | atccccagtc acaataccac caagagaccg ggttggcggc gcat | DNA |
| SEQ ID NO: 40 | Repe at-R | ctttccaata gctgcttgta gctgcggtta gtactgcaaa a | DNA |

**[Table 6]**

| | | | |
|---|---|---|---|
| SEQ ID NO: 41 | Right arm2-F | ttttgcagta ctaaccgcag ctacaagcag ctattggaaa g | DNA |
| SEQ ID NO: 42 | Right arm2-R | gcttaatgtg attgatctca aacttgatag | DNA |
| SEQ ID NO: 43 | HMGR -CF | gctgtctctg cgagagcacg tcga | DNA |
| SEQ ID NO: 44 | HMGR -CR | ggttcgcaca acttctcggg tggc | DNA |
| SEQ ID NO: 45 | BCO-s q1 | cttctgagta taagaatcat tcaa | DNA |
| SEQ ID NO: 46 | BCO-s q2 | tttgaaatat aaataacgtt ctta | DNA |

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Yarrowia lipolytica CC08-0125 | Accession number given by the
INTERNATIONAL DEPOSITARY AUTHORITY:
KCCM12972P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on April. 09. 2021 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: April. 09. 2021.

¹ Where Rule 6.4(d) applies, such date is the date on which the status of international depositary authority was
acquired.

Form BP/4 (sole page)
한국미생물보존센터 사단법인 한국종균협회
03641 서울특별시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yurim Bldg., 45, Hongjenae 2ga-gil, Seodaemun-gu, Seoul 03641, Korea. Tel: 82-2-391-0950, 396-0950 Fax: 82-2-392-2859

## Claims

1. A *beta*-carotene 15,15'-oxygenase variant, wherein the amino acid corresponding to position 43 from the N-terminus of an amino acid sequence of SEQ ID NO: 1, alanine; the amino acid corresponding to position 58 from the N-terminus of an amino acid sequence of SEQ ID NO: 1, leucine; and the amino acid corresponding to position 74 from the N-terminus of an amino acid sequence of SEQ ID NO: 1, isoleucine, are each substituted with a different amino acid.

2. The *beta*-carotene 15,15'-oxygenase variant of claim 1, wherein the variant comprises a substitution of the amino acid corresponding to position 43 from the N-terminus of an amino acid sequence of SEQ ID NO: 1 with an amino acid other than alanine; a substitution of the amino acid corresponding to position 58 from the N-terminus of an amino acid sequence of SEQ ID NO: 1 with an amino acid other than leucine; and a substitution of the amino acid corresponding to position 74 from the N-terminus of an amino acid sequence of SEQ ID NO: 1 with an amino acid other than isoleucine.

3. The beta-carotene 15,15'-oxygenase variant of claim 1, wherein the substitution of the amino acid corresponding to position 43 with a different amino acid is a substitution with a nonpolar amino acid; the substitution of the amino acid corresponding to position 58 with a different amino acid is a substitution with a polar amino acid; and the substitution of the amino acid corresponding to position 74 with a different amino acid is a substitution with a nonpolar amino acid.

4. The *beta*-carotene 15,15'-oxygenase variant of claim 1, wherein the different amino acid is valine or glutamine.

5. A polynucleotide encoding the variant of claim 1.

6. A microorganism comprising any one or more of the variant of claim 1 and a polynucleotide encoding the variant.

7. The microorganism according to claim 6, wherein the microorganism has an ability to produce a retinoid.

8. The microorganism according to claim 7, wherein the retinoid is any one or more selected from the group consisting of retinal, retinol, retinyl ester, and retinoic acid.

9. The microorganism according to claim 6, wherein the microorganism belongs to *Yarrowia* sp.

10. The microorganism according to claim 6, wherein the microorganism is *Yarrowia lipolytica.*

11. A method for producing a retinoid comprising culturing a microorganism comprising the variant of claim 1 or a polynucleotide encoding the variant in a medium.

12. The method of claim 11, further comprising recovering a retinoid from the medium or the microorganism.

13. The method of claim 11, wherein the microorganism belongs to *Yarrowia* sp.

14. The method of claim 11, wherein the retinoid is any one or more selected from the group consisting of retinal, retinol, retinyl ester, and retinoic acid.

15. A composition for producing a retinoid comprising the variant of claim 1, the microorganism of claim 8, or a culture of the microorganism.

16. Use of a microorganism for producing a retinoid comprising any one or more of the *beta*-carotene 15,15'-oxygenase variants, wherein the amino acid corresponding to position 43 from the N-terminus of an amino acid sequence of SEQ ID NO: 1, alanine; the amino acid corresponding to position 58 from the N-terminus of an amino acid sequence of SEQ ID NO: 1, leucine; and the amino acid corresponding to position 74 from the N-terminus of an amino acid sequence of SEQ ID NO: 1, isoleucine, are each substituted with a different amino acid; and a polynucleotide encoding the variant.
